# EUROPEAN PATENT APPLICATION

(11) **EP 4 796 533 A1**
(43) Date of publication of application: **26.08.2026**
(21) Application number: 24879209.5
(22) Date of filing: 18.10.2024
(51) Int. Cl.: C07D 317/36, B01J 27/22, B01J 21/06, B01J 35/73, C01B 32/921, C01B 32/90, C01G 23/00

(54) **METHOD FOR PRODUCING CYCLIC CARBONATES FROM CO2 BY USING MXENES AS CATALYSTS**

(30) Priority: 18.10.2023 ES 202330864
(71) Applicant: Universitat Politecnica de Valencia, 46022, Valencia (ES); AIMPLAS - Asociación de Investigación de Materiales Plásticos y Conexas, 46980 Paterna Valencia (ES)
(72) Inventor: GIMÉNEZ TORRES, Enrique, 46022 Valencia (ES); BENEDITO BORRÁS, Adolfo, 46980 Valencia (ES); BARJOLA RUIZ, Arturo, 46022 Valencia (ES); SOPEÑA DE FRUTOS, Sergio, 46980 Valencia (ES)
(74) Representative: Cueto, Sénida
(86) International application number: PCT/ES2024/070635
(87) International publication number: WO 2025/083313

(57) **Abstract**

The present invention relates to a process for obtaining cyclic carbonates that comprises reacting carbon dioxide with an epoxide in the presence of a MXene, using heterogeneous catalysis. The MXene is a 2D catalyst having a formula

Mₙ₊₁XₙT_{z}

wherein T represents surface termination groups (such as -OH, -F, -O, Cl or mixtures thereof),
n ranges from 1 to 4 both values inclusive,
z has a variable value, preferably, preferably 1 or 2,
M represents one or more transition metals and
X is carbon, nitrogen, or a mixture of both.

## Description

### Technical field of the invention

The present invention falls within the field of obtaining products of industrial interest such as carbonates, which also contribute to removing carbon dioxide from the atmosphere.

### Background

For some time now, transition metal carbides (TMCs), such as TiC, WC, MoC or Mo₂C, have attracted interest in the area of heterogeneous catalysis, either as active phases or as supports, because they possess catalytic properties similar of Pt group metals (Ru, Rh, Pd, Os, Ir and Pt), but their cost is significantly lower. This is particularly true for reactions involving the activation of C-H bonds in hydrocarbons, such as dehydrogenation, hydrogenation, or the conversion of methane to *syngas*. TMC applications also include electrocatalysis, in reactions such as the oxidation of H₂, CO and alcohols, or the reduction of O₂. Nevertheless, the practical application of TMCs in catalysis is very limited by their low surface area, typically < 5-10 m²g⁻¹. This situation changed radically with the discovery in 2011 of a new family of two-dimensional metal carbides, the MXenes [M. Naguib, M. Kurtoglu, V. Presser, J. Lu, J. Niu, M. Heon, L. Hultman, Y. Gogotsi, M. W. Barsoum, Adv. Mater. 23 (2011) 4248-4253], capable of achieving specific surfaces more than 10 times larger than conventional TMCs.

Two-dimensional (2D) transition metal-based carbides, nitrides, and carbonitrides, known as MXenes, are a new family of layered materials that has recently emerged due to their exceptional properties in terms of stability, electrochemical charge storage, electromagnetic shielding, filtration, and a wide range of additional applications.

MXene materials are obtained from layered precursors Mₙ₊₁AXₙ (n= 1, 2 and 3) called MAX phases, where "M" is a transition metal such as Ti, Zr, V, Cr, Ta, Nb, Mo; "A" is an element mainly from groups 13 or 14 (Si or Al), and "X" represents C and/or N [X. Wu, Z. Wang, M. Yu, L. Xiu, J. Qiu, Adv. Mater. 29 (2017) 1-8; F. Du, H. Tang, L. Pan, T. Zhang, H. Lu, J. Xiong, J. Yang, C. (John) Zhang, Electrochim. Acta 235 (2017) 690-699].

In this MAX structure the M-X bond exhibits ionic/covalent characteristics, while the M-A bond is purely metallic. Thus, the M-X bond is stronger than the M-A bond, allowing the A layer to be removed from the stratified solid.

By selectively removing the intermediate layers of element A from the MAX phase using a chemical etching process with suitable chemicals, a transition metal carbide/nitride called MXene is obtained, formed by stacked sheets with a certain degree of interlaminar separation, similar to the structure of graphene, whose formula can be expressed as Mₙ₊₁XₙT_{z} (where T represents surface termination groups such as -OH, -F, -O). Aqueous acidic solutions containing fluoride have generally been used to selectively attack the element A layers of MAX phases, either by using aqueous hydrofluoric acid (HF), or by *in situ* formation of HF through the reaction of hydrochloric acid (HCl) and a fluoride (e.g., LiF).

After acid attack, the multilayer MXene can be exfoliated or delaminated into aqueous colloidal suspensions, improving the efficient utilization of the 2D surface of the monolayer nanosheets for later applications. However, the degree of exfoliation will depend on the conditions used during the chemical attack.

In those cases where a fluoride-based compounds such as HCl/LiF is used, the Li⁺ cations are intercalated between the MXene sheets, facilitating their exfoliation by simple shaking or sonication. However, when HF is used as an acidic medium, exfoliation is only possible through the intercalation of organic molecules or ions between the layers, as it increases the separation between the layers and weakens the interlayer interactions, facilitating their subsequent exfoliation. However, it should be noted that due to the use of chemical attack agents, the surface of MXene can be coated with different functional groups because it is chemically active. Intrinsically metallic MXenes can become semiconductors, as a consequence of the presence of T surface terminations, such as -F or -OH.

MXenes not only have a 2D layered structure, but also possess hydrophilic surface properties, high mechanical and chemical stability, and good electrical conductivity. Furthermore, they can contain many different cations between its layers. These exceptional properties have focused interest in different fields, such as energy storage, fuel cells, adsorption processes, or sensors. Significant advances have also been made in areas such as spintronics, portable electronic devices, environmental pollutant removal, and biomedicine.

On the other hand, considering the catalytic applications of TMC and the high reactivity and specific surface area of MXenes, significant development of this new family of materials is expected in the field of catalysis. However, the application of MXenes in conventional (thermal) heterogeneous catalysis has been much less investigated, and the results are basically limited to 5 reactions: 1) Oxidation of CO to CO₂; 2) Activation and conversion of CO₂; 3) water-gas shift. CO + H₂O → CO₂ + H₂; 4) Fixation of N₂ and synthesis of NH₃; and 5) Dehydrogenation of alkanes (ethylbenzene to styrene) and hydrodeoxygenation of oxygenated compounds (anisole or guaiacol to phenol). Therefore, in most cases, these are activation reactions of small molecules in the gas phase and under conditions of high temperature and pressure. However, to date there are no catalytic studies of the conversion of complex molecules under mild reaction conditions (liquid phase, atmospheric pressure and temperatures below 100-120°C), which have a much lower energy demand and allow the preparation of more complex molecules typical of Fine Chemistry or Pharmaceutical Chemistry.

The possibilities offered by CO₂ as a raw material are many. Removing it from the atmosphere and reusing it has become a necessary formula to achieve the emissions reduction goals of the Paris Agreement, also contributing to decarbonizing sectors as important as mobility.

Each year, less than 2% of the 7.5 billion tons of CO₂ produced is used by industry; the remaining 98% represents a business opportunity for the chemical and biorefinery industries.

The low reactivity of the CO₂ molecule makes it necessary to require catalyzed processes to transform it. Among the various catalyzed processes, the synthesis of cyclic carbonates from CO₂ is one of the most promising methodologies. This reaction involves reacting CO₂ with epoxides in the presence of catalysts. Among the various reaction products, cyclic carbonates and polycarbonates can be obtained, both with a high added value.

Cyclic carbonates are important industrial chemicals that have diverse applications: environmentally friendly solvents, in lithium-ion batteries, paints and coatings, resins, precursors of polymeric materials and polymer processing in fine chemicals. The two most industrially used cyclic carbonates are ethylene carbonate and propylene carbonate, but there are other more specialized ones that also have high commercial interest.

Other products of great interest are polycarbonate resins, which make up a group of thermoplastic materials widely used today. They are commonly used to manufacture plastic products, such as bottles, sports glasses, etc. These polymers can also be obtained by copolymerization between the CO₂ molecules and epoxides in the presence of an appropriate catalyst. CO₂ is used as a raw material in the manufacture of these polymers, being incorporated up to 50% into their molecular structure.

Other polymers synthesized from CO₂ and also widely used are polypropylene carbonate-based polymers that incorporate around 20% CO₂ in their structure. Its demand is increasing worldwide. These polyols have a high added value by contributing to the reduction of the greenhouse effect and can be used for the manufacture of adhesives, paints and coatings, elastomers, wood composites and medical equipment, among others.

Isocyanate-free polyurethanes are other polymers synthesized from CO₂. Alternative synthetic routes for obtaining non-isocyanate polyurethanes (NiPUs) are based on the cycloaddition of CO₂ to an epoxide, resulting in cyclic carbonates as chemical intermediates.

The present invention addresses a novel strategy for heterogeneous catalysts based on 2D morphologies. MXenes, with their structural characteristics, present a broad potential for catalyzing CO₂ conversion reactions into cyclic carbonates.

In summary, CO₂ allows the replacement of other toxic substances such as phosgene or isocyanates to obtain high value-added products such as cyclic carbonates, polycarbonates and polyurethanes. The use of heterogeneous catalysts based on MXenes achieves greater efficiency in conversion reactions.

The coupling reaction of CO₂ with epoxides to obtain carbonates is one of the most studied in the chemistry of CO₂ transformation. The interest lies in the multiple applications of the organic cycle carbonates obtained and in the simplicity of the process if active, selective, stable and recoverable catalysts are available. Essentially, transition metal catalysts are used in homogeneous processes.

Heterogeneous catalysts can be considered those solid catalysts that are used in chemical reactions whose reactants and products are in a liquid and/or gaseous phase, that is, in a different phase than the catalysts.

In heterogeneous catalysts, the catalytic phenomenon is related to the chemical properties of the surface of the solid catalyst. For it to be effective, one or more of the reagents must be chemisorbed onto it, giving rise to a substrate-catalyst complex that will cause the decrease in activation energy. Physical adsorption is important in heterogeneous catalysis only in cases such as radical recombination.

Heterogeneous catalysts effectively overcome some of the problems of homogeneous catalysts. In particular, a much less complex synthesis process, more affordable costs, much simpler separation and subsequent purification of the catalyst, called regeneration, long catalyst life, among others.

Some documents that show the use of MXenes in CO₂ utilization reactions are, for example, WO/2020/005482 which discloses the conversion into hydrocarbons by the reaction catalyzed by MXene of transitions metals.

Another document that discloses epoxide conversion reactions by catalysis with MXenes is Slot T K; Rothenberg G; Shinju N R; Natu V; Barosum M; Ramos-Fernandez E V; Sepulveda-Escribano A: "Enhancing catalyric epoxide ring-opening selectivity using surface-modified Ti3C2Tx Mxenes" 2D Mater. 8 (2021), which shows a study of the kinetics and mechanism of the epoxide ring-opening reaction catalyzed with modified MXenes. It is shown that the kinetics and selectivity in the opening of the epoxide ring can be controlled and enhanced by modifying the surface of the Ti₃C₂Tₓ, which influences the number and type of acid sites.

Furthermore, it is shown that by oxidizing MXene to form a Ti₃C₂Tₓ-TiO₂ compound, the catalytic activity increases.

Another document that discloses applications of MXenes as heterogeneous catalysis is Morales-Garcia A; Calle-Vallejo F; Illas F: "New Horizons in Catalysis" ACS Catal. 2020, 10, 22, 13487-13503, in which CO₂ activation reactions are mentioned, but among whose applications the formation of carbonates is not included.

The document "2D Early Transition Metal Carbides (MXenes) for Catalysis", Small 2018, 1804736, doi.org/10.1002/smll.201804736 is a review published in 2019 of possible application of MXenes. These are reviewed in section "4 MXene-Based Catalysts for Conventional Heterogeneous Catalysis" of applications as a heterogeneous catalyst. However, the applications collected do not include the use of CO₂ and epoxides as precursors in the formation of carbonates.

Other reviews found relating to the applications of MXenes include, for example: L. Verger, V. Natu, M. Carey, M. W. Barsoum "MXenes: An Introduction of Their Synthesis, Select Properties, and Applications", Trends in Chemistry, Volume 1, (7) 656-669 (2019), doi.org/10.1016/j.trechm.2019.04.006.

### Description of the invention

The present invention relates to a process for obtaining carbonates comprising reacting carbon dioxide with an epoxide in the presence of a MXene, by heterogeneous catalysis.

The present invention relates, more particularly, to a process for obtaining cyclic carbonates comprising reacting carbon dioxide with an epoxide in the presence of an MXene, by heterogeneous catalysis, wherein the MXene is a 2D catalyst having a formula.

Mₙ₊₁XₙT_{z}

wherein T represents surface termination groups selected from -OH, -F, -O, Cl and mixtures thereof,
n ranges from 1 to 4 both values included,
z has a value of between 1 and 10,
M represents one or more transition metals selected from Sc, Ti, Zr, Hf, V, Nb, Ta, Cr, Mo, Co, and combinations thereof and
X is carbon, nitrogen, or a mixture of both.
z has a variable value, depending on the predominant functional group. For example, in C. Lei, X. Zhang, Z. Zhou, Recent advances in MXene: preparation, properties, and applications, Front. Phys. 10 (2015) 276e286. DOI 10.1007/s11467-015-0493-x, has a value of 1 or 2, or in Naguib, M., Kurtoglu, M., Presser, V., Lu, J., Niu, J., Heon, M., ... Barsoum, M. W. (2011). Two-Dimensional Nanocrystals Produced by Exfoliation of Ti3AlC2. Advanced Materials, 23(37), 4248-4253. doi:10.1002/adma.201102306 has a value of 1 or 2.

Therefore, "z" can have a value between, for example, 1 and 10, preferably between 1 and 10, and more preferably between 1 and 5, and even more preferably 1 or 2.

The index n determines the thickness of the layer. The functional groups on the T surface are generally a mixture of O, -OH, F and/or Cl in the most common cases.

These T groups play a decisive role in many aspects, as they are responsible for the interactions between the different MXene layers and play an important role in the interactions between the MXene sheets and different environments, such as gases or other functional molecules/materials, which are key to the design of advanced compounds.

Since the T groups are directly bonded to the external M elements, the T groups can drastically modify the electronic structure and properties of the MXene layers, with the possibility, for example, of inducing superconductivity or controlling its magnetic state.

Due to the enormous possibilities offered by surface finishes to modify the properties and expand the field of application of MXenes, great efforts have been devoted to the chemical modification of the surface of MXenes, with the ultimate goal of being able to control it.

This control can be achieved through the exfoliation process, as it has a strong impact on the different T terminal groups that can be generated for a given MAX precursor. Likewise, control can also be achieved through the application of subsequent treatments, such as thermal annealing or reaction in the presence of several atmospheres.

The quantitative characterization of the surface chemistry of the MXene sheets still appears to be an important problem and one that still needs to be better understood. It has been approached using different techniques such as X-ray photoelectron spectroscopy (XPS), transmission electron microscopy and Raman spectroscopy, among others.

In general terms, the quantification of surface groups obtained for similar MXenes may in principle, vary substantially depending on the synthesis method used. There are many possible reasons for this, including differences in chemical synthesis/drying protocols or MAX phase precursors, resulting in MXenes with different surface groups.

The MXenes according to the invention comprise at least one transition metal, and preferably the transition metals are selected from Sc, Ti, Zr, Hf, V, Nb, Ta, Cr, Mo, Co and combinations thereof.

"X" in the process of the invention may be C or N, or both, and is preferably C.

MXenes are obtained from layered precursors Mₙ₊₁AXₙ (n = 1, 2 or 3) called "MAX phases", where "M" is a transition metal as defined; "A" is an element from groups 13 or 14 of the periodic table (e.g., Si or Al). The procedure for preparing MXenes is known. Some references on MXenes synthesis are mentioned in the Examples section.

An "epoxide" according to the invention can be a monocyclic, dicyclic or tricyclic epoxide. Examples of monocyclic epoxides are epoxyethane, 2,3-epoxyhexane, 1,2-epoxycyclohexane, 1,2-epoxypropane.

Examples of diepoxides are bisphenol A diglycidyl ether (DGEBA), diepoxides with alkyl groups up to 10 carbon hydrocarbon chains between the two tricycles, for example, 1,3-butadienediepoxide, 1,5-hexadienediepoxide, 1,7-octadienediepoxide.

Examples of epoxides are trimethylolpropane triglycidyl ether (TMPTE), and other naturally occurring epoxides derived from myo-inositol.

The molar ratio of catalyst: epoxide can be between 1:5 and 1:200, preferably between 1:5 and 1:1000.

In the process of the invention, cocatalysts can be used, such as a quaternary ammonium cationic surfactant that acts as a surfactant, preventing the agglomeration of the main heterogeneous catalyst and improving its morphology and interaction with the epoxide.

An example of these cationic surfactants is cetyltrimethylammonium (CTAB). CTAB is a micellar co-catalyst that generates micelles in solution, allowing the growth (i.e., the yield) of the carbonate. Another example of a surfactant is SDS (sodium dodecyl sulfate) or lauric acid.

The molar ratio of co-catalyst: epoxide can be between 1:5 and 1:200, preferably between 1:5 and 1:100.

The reaction between carbon dioxide and epoxide, according to the process of the invention, can be carried out under CO₂ pressure, for example, between 5 and 50 bar, preferably between 10 and 30 bar.

The reaction according to the process of the invention can be carried out, for example, between 30 and 150°C, preferably between 80 and 130°C.

The reaction according to the process of the invention is carried out under constant stirring.

The reaction according to the procedure of the invention is carried out with or without solvent.

Any polar solvent can be used as a solvent in the process of the invention, for example, dimethyl sulfoxide (DMSO), dimethylformamide (DMF), hexamethylphosphoramide (HMPA).

Scheme 1 shows a particular embodiment according to the process of the invention, in which 1,3-butadienediepoxide is transformed into butadienedicarbonate without the need to use solvents, and "cat/co-cat" means "catalyst/co-catalyst". In this case, the reaction can be carried out in the absence of solvent, since the carbonate obtained can be used for other reactions and products. 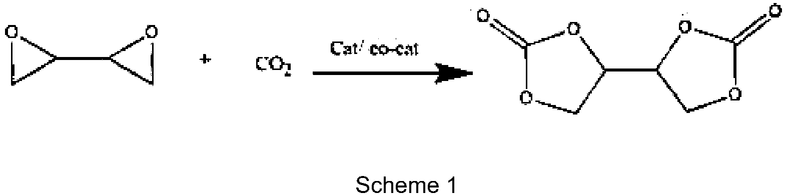
cat: catalyst
Co-cat: cocatalyst

Scheme 2 shows a singular embodiment according to the process of the invention, in which the triepoxide called trimethylolpropane triglycidyl ether (TMPTE) is transformed with CO₂ and with the catalysts of the invention into trimethylolpropane tris-carbonate (TMPTC). In this case, the reaction can be carried out in the absence of solvent, since the carbonate obtained can be used for other reactions and products.

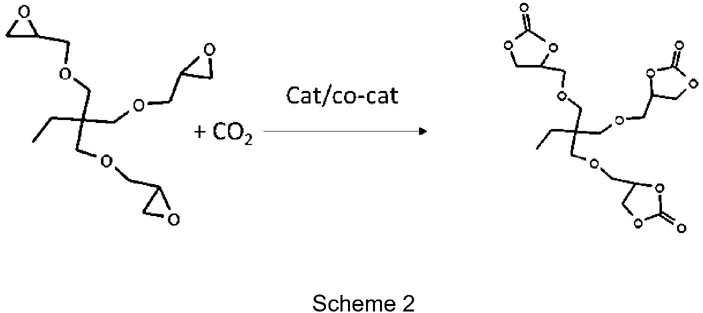

According to a preferred alternative, the heterogeneous catalyst is Ti₃C₂.

A further particular embodiment relates to the process of the invention, comprising an amination step of the corresponding cyclic carbonate resulting in the production of non-isocyanate polyurethanes.

If carbonation and subsequent amination were to be carried out in a "one-pot", any polar solvent, for example, any of those mentioned above would be useful.

To prevent the presence of moisture, desiccants such as silica gel can be used.

The process of the invention may include the use of hybrid Mxenes, i.e. with two or more transition metals, such as: Mo₂Ti₂C₃Tₓ; Cr₂TiC₂Tₓ; Mo₄VC₄Tₓ; Ti₂-_{y} Nb_{y} CTₓ (DOI 10.1088/2053-1583/ac4ba2).

### BRIEF DESCRIPTION OF THE FIGURES

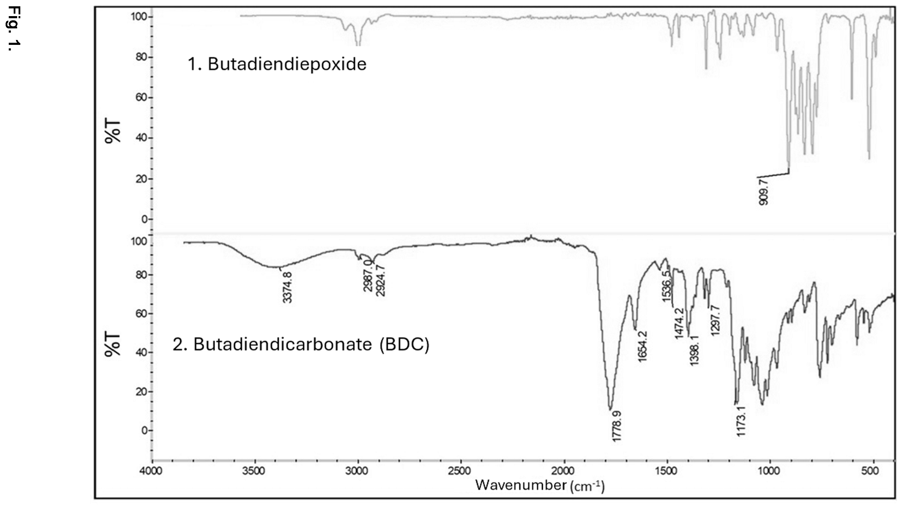

FIGURE 1: FTIR spectra for the reagent 1,3-butadienediepoxide (top) and for the product butadienedicarbonate or BDC (bottom).

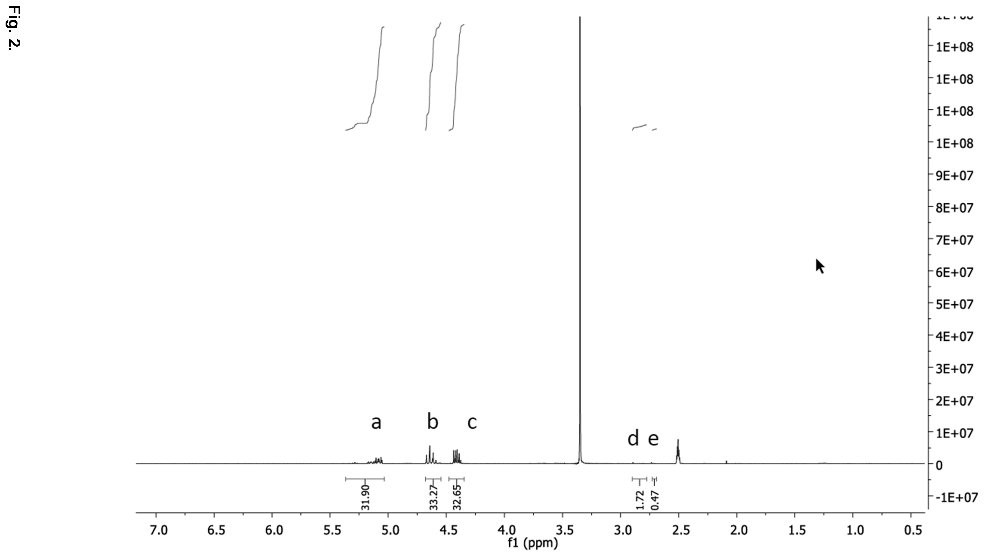

FIGURE 2: Integrated ¹H-NMR spectrum for the reaction product 1,3-butadienediepoxide (signals d and e) with CO₂, to obtain butadienedicarbonate (signals a, b and c). The peaks corresponding to the methylene and methine protons near the epoxy group in 1,3-butadienediepoxide, which appear at 2.68, 2.79 and 2.85 ppm, disappear after the coupling reaction and new peaks appear at δ= 4.25-4.47 (m, 2 H, 1- CH2), 4.60-4.70 (t, 8.8Hz, 2 H, CH2), 5.04-5.13 (m, 2 H, CH) ppm.

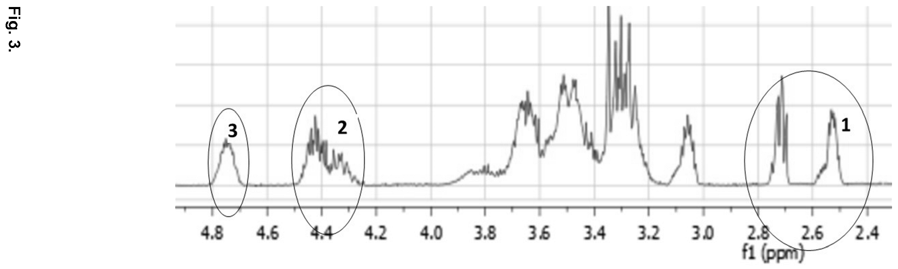

FIGURE 3: Spectrum ¹H-NMR of a tricyclic carbonate obtained from the reaction of CO₂ with Trimethylolpropane triglycidyl ether (TMPTE). The signals labeled 1 correspond to the protons of the epoxide groups, while 2 and 3 correspond to those of the cyclic carbonates (protons corresponding to CH3 and CH2, respectively).

### EXAMPLES

### EXAMPLE 1

The starting MXenes can be prepared by a conventional procedure, as described in: J.C. Lei, X. Zhang, Z. Zhou, Recent advances in MXene: preparation, properties, and applications, Front. Phys. 10 (2015) 276e286. DOI 10.1007/sl 1467-015-0493-x, or in
J. Yang, B. Chen, H. Song, H. Tang, C. Li, Synthesis, characterization, and tribological properties of two-dimensional Ti3C2, Cryst. Res. Technol. 49 (2014). 926-932. DOI 10.1002/crat.201400268, or in
Lim, Kang Rui Garrick, Mikhail Shekhirev, Brian C. Wyatt, Babak Anasori, Yury Gogotsi, Seh Zhi Wei. "Fundamentals of MXene synthesis." Nat. Synth 1, no. 8 (2022): 601-614. DOI: 10.1038/ s44160-022-00104-6

An example of MXene synthesis is a procedure as described below: Initially, 1 gram of Ti₃AlC₂ was gradually added to 10 ml of a 40% HF solution in water. The mixture was left under magnetic stirring at room temperature for 18 hours. The dispersion obtained was centrifuged at 8000 rpm, after which the supernatant was removed and washed with distilled water bubbled with Argon. This process was repeated until the pH of the supernatant liquid reached a value of 6.

The acid-free compound, labeled *Ti₃C₂Tₓ,* was subsequently subjected to a freeze-drying process. Once dry, it was stored in darkness at a temperature of 4°C.

An example of the process of intercalation of cations Li⁺ and subsequent delamination of the product Ti₃C₂Tₓ is described below: 50 mg of dry Ti₃C₂Tₓ was added to 10 ml of a 500-ppm solution of DDAC (didecyldimethylammonium chloride) in water, keeping it under stirring for 18 hours. Subsequently it was centrifuged at 8000 rpm, discarding the supernatant, and the material deposited at the bottom of the vial was redispersed with 50 ml of distilled water bubbled with argon, forming a dispersion of 1 mg/ml. This dispersion was sonicated for 6 hours in an ultrasonic bath. Finally, it was filtered with a 0.22-micron membrane filter and washed with water to remove any remaining DDAC, then subjected to a freeze-drying process.

The review article titled Progresses and Challenges in 2D MXenes: Synthesis, Intercalation/Delamination, and Storage, Nasima Khatun; Mahapatra and Singh; Age of MXenes, Volume 1. Fundamentals and Artificial Intelligence: Machine Learning Interventions ACS Symposium Series; American Chemical Society: Washington, DC, 0 mentions other possible intercalation agents, such as urea, hydrazine monohydrate, dimethylformamide, or dimethyl sulfoxide.

### Obtaining butadiene dicarbonate from 1,3-butadiene diepoxide

### 2. Materials.

The materials used during the reaction were as follows: 1,3-butadienediepoxide (97%), dimethyl sulfoxide (DMSO-d6) as a solvent, silica gel 60 as a desiccant (Munich, Germany), and hexadecyltrimethylammonium bromide (CTAB, >98%), all from SIGMA-ALDRICH. Pure CO₂ was supplied by LINDE (Valencia, Spain), while the MXene-based catalysts were synthesized by the Universitat Politècnica de València, according to the method described in the references mentioned at the beginning of this section. The acetone was supplied from VWR Chemicals (Spain). In all cases, MXene Ti₃C₂Tx was used, where Tx are surface modifications that can be -OH, -H, or -F.

### 3. Experimental Setup and Process.

For the experimental carbonation reaction, a pressurized, autoclave-type reactor made of stainless steel (MiniClave, 190 ml, type Br-100, Berghof) was used, equipped with an internal cavity or bed of Teflon^{®} and an "O-ring".

The reactor consists of a gas inlet or feed, through which the gas purges and the final CO₂ load necessary for the reaction were carried out. The installed pressure gauge determines the CO₂ pressure at all times, which is a clear indicator of its consumption and the evolution of the reaction. The liquid sample extraction point allows the system to be depressurized once the reaction is complete. An external thermocouple is introduced to control the temperature during the evolution of the carbonation process.

In all the reactions studied, 2 ml of 1,3-butadienediepoxide were added to the reactor, as well as a constant amount of Ti₃C₂Tx catalyst prepared as indicated in section 1 above and at the beginning of this "Examples" section. The molar ratio has been 1:1000, catalyst:diepoxide. The co-catalyst used (CTAB) was also introduced with the same molar ratio as the heterogeneous catalyst itself, i.e., 1:1000.

The "experimental procedure" in all cases mentioned in Table 1 was as follows: initially a Teflon tube was filled with the 1,3-butadieneepoxide and the corresponding catalyst/co-catalyst. After the reactor was properly closed, it was purged three times with 30 bar of CO₂. The reactor was then left pressurized to 30 bar with CO₂. The reactor heated up to 120°C causing an immediate increase in pressure. Once the working temperature was reached, the pressure of CO₂ was 40-45 bar. The reaction took place under constant stirring (650 rpm).

After the set reaction time, the reactor was placed in an ice bath until it reached room temperature, which is required to proceed with a slow and controlled depressurization that would allow the dissolved CO₂ to leave the carbonated product.

The conversion reaction of diepoxide with CO₂ was studied by NMR (nuclear magnetic resonance) analysis and infrared spectroscopy (FTIR) of the reaction crude.

Infrared spectroscopy studies were performed using a Nicolet 6700 FT-IR spectrometer (Thermo Scientific, Illinois, USA) with a diamond tip (30,000-200 cm⁻¹).

The proton nuclear magnetic resonance was determined at a frequency of 300 MHz and room temperature.

The materials obtained were adequately purified in a conventional chromatographic separation column. For this purpose, as an example, in the case of the synthesis of butadiene dicarbonate, preliminary work was carried out using thin layer chromatography or TLC, in order to select the most appropriate eluent for the separation of the products. Ethyl acetate/hexane ratios of 2:1 and 5:1 were suitable for separating products from unreacted reagents and co-catalyst. The separation column was loaded with a mixture of silica and eluent.

To recover the catalyst, a pre-filtration was sufficient, especially in cases of high conversion level (>95%), where the separation column was not necessary.

### 4. Reactions performed and results.

Studies conducted with two types of synthesized MXenes:
MX-1: MXene Ti₃C₂Tₓ obtained in the synthesis as described in Example 1, and which was not subsequently subjected to any centrifugation. MX-1 is obtained without subsequent delamination.
MX-2: MXene Ti₃C₂Tₓ obtained in the synthesis as described in Example 1. Subsequently, the product obtained was subjected to an intercalation process with DDAC in order to achieve greater delamination of the lamellae. MX-2 is obtained delaminated with DDAC (HF).

The efficiencies of MXenes in the carbonate production process of the invention have been compared with other latest generation heterogeneous catalysts, such as MOFs ("Metal Organic Frameworks"), which have been extensively studied in the relevant literature.

**Table 1. Reaction yields for obtaining BDC (butadiene epoxide carbonate) with MOF catalysts and with MXenes at constant conditions of 120°C and 30 bar pressure (using CTAB as a co-catalyst/dispersant).**

| Catalyst | Reaction time (hours) | NMR yield (%) |
|---|---|---|
| Co-MOF-74 | 6 | 65 |
| Al-PDA* | 6 | 73 |
| Al-OH-fumarate** | 6 | 95 |
| MX-1 | 6 | 100 |
| MX-2 | 6 | 100 |
| Al-OH-fumarate (without co-catalyst) | 6 | 5 |
| MX-1 (without co-catalyst) | 6 | 42 |
| Reference: CTAB only (co-catalyst) | 6 | 35 |
| Al-OH-fumarate | 6 | 35 |
| Al-OH-sumarate | 3 | 1-2 |
| MX-1 | 3 | 100 |
| MX-2 | 3 | 91 |
| Reference: CTAB only (co-catalyst) | 3 | 3-5 |
| MX-1 | 1.5 | 58 |
| MX-2 | 1.5 | 51 |

| | | |
|---|---|---|
| *AI-PDA: MOF based on Al-Polydopamine; **AI-OH-fumarate: MOF. It is a MOF ("Metal Organic Framework") type catalyst that has already been used previously, as cited in: A. Benedito, E. Acarreta, E. Giménez "Highly Efficient MOF Catalyst Systems for CO2 Conversion to Bis-Cyclic Carbonates as Building Blocks for NIPHUs (Non- Isocyanate Polyhydroxyurethanes) Synthesis" Catalysts, 11, 628 (2021). DOI .org/10.3390/catal 11050628 | | |

From this table it can be observed that these catalysts greatly improve the efficiency of the reaction at short reaction times. In 3 hours of reaction they continue to maintain high levels of conversion, especially when compared to other highly active heterogeneous catalysts such as MOFs ("Metal Organic Frameworks"). The blanks with CTAB (co-catalyst) showed somewhat reduced catalytic activity (at 6 hours of reaction), thus demonstrating the synergistic effect of CTAB with specific heterogeneous catalysts, whether MOFs or MXenes. However, their efficiency at short reaction times (1-3 hours) drops critically, both with CTAB and MOFs, while MXene maintains it.

The product of each reaction was analyzed and identified using FTIR, and an example of this is shown in Figure 1.

The calculation of the yields starts from the integration of the ¹H-NMR signal peaks, as can be seen in the reaction product example in Figure 2. The chemical changes of the methylene and methine protons of the epoxy group in the 1,3-butadienediepoxide at 2.68, 2.79 and 2.85 ppm disappear after the coupling reaction and new peaks appear at at δ= 4.25-4.47 (m, 2 H, 1-CH₂), 4.60-4.70 (t, 8.8Hz, 2 H, CH₂), 5.04-5.13 (m, 2 H, CH) ppm.

### EXAMPLE 2

### Triple carbonate

Obtaining tricyclic carbonates of the type "Trimethylolpropane triglycidyl carbonate" (TMPTC) from the reaction of CO₂ with Trimethylolpropane triglycidyl ether (TMPTE), a trifunctional aliphatic glycidyl ether epoxide monomer. The experimental procedure 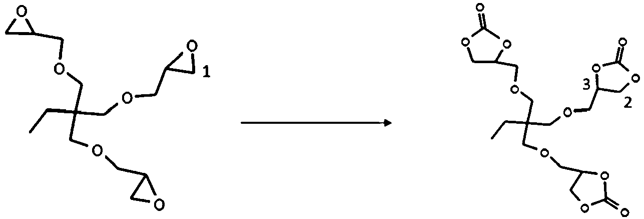 was identical to that described in example 1

The MXenes used were the same as in example 1.

The conversion reaction of triepoxide with CO₂ was studied using NMR (nuclear magnetic resonance) analysis. The resonance spectrum is shown in Figure 3. We calculate the performance using the area ratio of the following NMR spectrum signals:
Using the absolute value of the area under the integral, we obtain the percentage. $Yield = \frac{Area 2 + Area 3}{Area 1 + Area 2 + Area 3} \times 100$

And the results are shown in the following table:

| **TMPTE (g)** | **Catalyst CTAB (G)** | **MX1 (g)** | **MX2 (g)** | **Time (min)** | **T (°C)** | **Yield** |
|---|---|---|---|---|---|---|
| 17.58 | 1.03 | X | X | 90 | 130 | 100% |
| 18.22 | 1.01 | X | X | 30 | 130 | 96.67% |
| 17.56 | 0.99 | 0.01694 | X | 90 | 70 | **56.36%** |
| 17.78 | 1.04 | X | 0.01758 | 90 | 70 | **45.60%** |
| 17.78 | 1.04 | X | X | 90 | 70 | 39.68% |
| 18.01 | 0.99 | 0.01747 | X | 90 | 60 | 17.06% |
| 17.51 | 1.01 | X | 0.01732 | 90 | 60 | 16.58% |
| 17.89 | 1.03 | X | X | 90 | 60 | 16.98% |

| | | | | | | |
|---|---|---|---|---|---|---|
| X = without MXene addition | | | | | | |

The critical factor in the reaction is temperature; below 70 °C the reaction takes place very inefficiently. It can be observed that in the absence of MXenes the reaction has a much lower yield.

The use of a small amount of MXene along with the CTAB co-catalyst improves the reaction's performance. Between the two MXenEs studied, MX1 shows better performance.

## Claims

1. A process for obtaining cyclic carbonates comprising reacting carbon dioxide with an epoxide in the presence of an MXene, by heterogeneous catalysis, wherein the MXene is a 2D catalyst having a formula
Mₙ₊₁XₙT_{z}
wherein T represents surface termination groups selected from -OH, -F, -O, Cl and mixtures thereof,
n ranges from 1 to 4 both values inclusive,
z has a value of between 1 and 10,
M represents one or more transition metals selected from Sc, Ti, Zr, Hf, V, Nb, Ta, Cr, Mo, Co, and combinations thereof and
X is carbon, nitrogen, or a mixture of both.

2. The process according to claim 1 wherein the epoxide is a monocyclic, dicyclic or tricyclic epoxide.

3. The process according to claim 2, wherein the epoxide is a monocyclic epoxide selected from epoxyethane, 2,3-epoxyethane, 1,2-epoxycyclohexane and 1,2-epoxypropane.

4. The process according to claim 2, wherein the epoxide is a bicyclic epoxide selected from bisphenol A diglycidyl ether (DGEBA), diepoxides with alkyl groups up to 10 carbon hydrocarbon chains between the two tricycles.

5. The process according to claim 2, wherein the epoxide is selected from trimethylopropane triglycidylether (TMPTE) and a myo-inositol-derived epoxide.

6. The process according to any one of claims 1 to 5, comprising the use of cocatalysts.

7. The process according to the preceding claim, wherein the cocatalyst is a surfactant, preferably a quaternary ammonium cationic surfactant, preferably a quaternary ammonium cationic surfactant, and more preferably cetyltrimethylammonium (CTAB).

8. The process according to any one of claims 1 to 7, wherein the catalyst and the epoxide are in a molar ratio between 1:5 and 1:200, preferably between 1:5 and 1:100 by weight.

9. The process according to any of claims 1 to 8 comprising reacting 1,3-butadienediepoxide with CO₂.

10. The process according to any of claims 1 to 8, comprising reacting trimethylolpropane triglycidyl ether with CO₂.

11. The process according to any of the preceding claims, comprising an additional step of amination of the corresponding cyclic carbonate resulting in non-isocyanate polyurethanes.
